Europäisches Patentamt

European Patent Office

Office européen des brevets

(19)

(11) Numéro de publication: **0 250 300 B1**

(12) **FASCICULE DE BREVET EUROPEEN**

(45) Date de publication de fascicule du brevet:
28.08.91

(51) Int. Cl.5: **A61K 7/06**, A61K 7/48,
A61K 33/30, A61K 31/44

(21) Numéro de dépôt: **87401319.6**

(22) Date de dépôt: **12.06.87**

(54) **Utilisation des mélanges de sel de zinc et de la vitamine B6 pour enrayer les processus alopéciques et/ou séborrhéiques.**

(30) Priorité: **16.06.86 FR 8608670**

(43) Date de publication de la demande:
**23.12.87 Bulletin 87/52**

(45) Mention de la délivrance du brevet:
**28.08.91 Bulletin 91/35**

(84) Etats contractants désignés:
**BE CH DE FR GB IT LI LU NL**

(56) Documents cités:
EP-A- 0 048 473      WO-A-84/04243
DE-A- 3 140 954      FR-M- 3 203
GB-A- 2 106 386      US-A- 4 161 526

(73) Titulaire: **SOCIETE BFB**
**7, rue Michelet**
**F-93360 NEUILLY PLAISANCE(FR)**

(72) Inventeur: **Bigou, Alphonse**
**60, Avenue Paul Doumer**
**F-75016 Paris(FR)**

(74) Mandataire: **Combe, André et al**
**CABINET BEAU DE LOMENIE 55, rue d'Ams-**
**terdam**
**F-75008 Paris(FR)**

## Description

La présente invention concerne l'utilisation du zinc et de la vitamine B$_6$ pour enrayer les processus alopéciques et/ou séborrhéiques.

Les androgènes circulants, d'origine testiculaire (testostérone), ovarienne et surrénalienne, sont, en tant que tels, inactifs au niveau du follicule pilo-sébacé.

Ils ne deviennent actifs, au niveau de celui-ci, devenu alors organe-cible, qu'après leur transformation en un métabolite, la dihydrotestostérone (DHT), sous l'effet d'une enzyme, la 5-α-réductase.

L'équipement enzymatique en 5-α-réductase des follicules pilosébacés peut être exagéré, par prédisposition génétique, chez certains sujets et/ou au niveau de certaines zones du cuir chevelu.

Dès lors, l'excessive production locale de DHT va stimuler l'un et/ou l'autre pôle d'activité du follicule pilo-sébacé :
- le pôle pilaire où la DHT enclenche un processus involutif perturbant le cycle pilaire et aboutissant à l'alopécie,
- la glande sébacée où la DHT provoque une hypertrophie et une hyperséborrhée.

Il a été trouvé que le zinc, sous forme de sel soluble, associé à la vitamine B$_6$ pouvait constituer des mélanges très efficaces pour réduire l'activité de la 5-α-réductase et par conséquent s'opposer aux processus alopéciques et/ou séborrhéiques dans la mesure où lesdits mélanges :
- contenaient des quantités relatives de zinc et de vitamine B$_6$ telles que cette dernière potentialise l'action du zinc,
- étaient appliqués topiquement.

La présente invention concerne donc les compositions cosmétiques et pharmaceutiques utilisables par application sur la peau et contenant du zinc, généralement sous forme d'un sel et de la vitamine B$_6$, la proportion de ces deux constituants étant telle qu'il y a potentialisation de l'activité anti-5-α-réductase du zinc par la vitamine B$_6$.

Les compositions selon l'invention contiendront donc :
- de 0,004 à 2 % en poids, exprimé en zinc, d'un sel de zinc,
- de 50 à 1000 % en poids, par rapport au zinc, de vitamine B$_6$.

Lesdites compositions sont bien évidemment conditionnées de façon que le zinc présent soit acheminé au niveau des follicules pilo-sébacés ; ainsi lorsque le sel de zinc est utilisé dans un milieu aqueux les conditions dudit milieu doivent être telles que ledit sel y soit soluble.

Lesdites compositions pourront être utilisées à la fois sur toute surface de la peau présentant un excès de séborrhée et (plus particulièrement) sur le cuir chevelu où l'action desdites compositions peut être à la fois contre l'excès de séborrhée que l'on détecte souvent à ce niveau et contre les autres phénomènes entraînant la chute des cheveux.

Selon un second aspect, la présente invention vise à couvrir l'utilisation d'une composition comportant de 0,004 à 2 % en poids, exprimé en zinc, d'un sel de zinc, de 50 à 1000 % en poids, par rapport au zinc, de vitamine B$_6$, pour la préparation d'une composition pharmaceutique destinée à la lutte contre les processus alopéciques et/ou séborrhéiques.

Selon une caractéristique particulière, le sel de zinc précité est soluble dans l'eau.

On a déjà préconisé comme produit anti-chute pour les cheveux des compositions à base de méthionine et qui peuvent contenir comme adjuvants soit de la vitamine B$_6$, soit du sulfate de zinc, soit un mélange de ces deux produits. Par rapport à cet art antérieur, la présente invention, venant de ce qu'un nouveau mode d'activité du zinc a été mis en évidence, se caractérise donc essentiellement en ce que les compositions revendiquées ne comportent pas de méthionine.

On a également revendiqué, dans le brevet européen 48473, de nouvelles compositions pharmaceutiques contenant un sel de zinc et de 0,5 à 1,5 parties en poids, pour une partie en poids de zinc d'acide glutamique. Il est indiqué dans ce brevet que lesdites compositions peuvent contenir également de la vitamine B$_6$ et peuvent être utiles pour la régénération de certaines atrophies de la peau incluant la réponse des cheveux [lorsque lesdites atrophies en sont la cause].

On notera cependant :
- que les compositions du brevet européen 48473 doivent nécessairement contenir de l'acide glutamique qui est absent des compositions selon l'invention,
- que les compositions du brevet européen 48473 sont utilisables par voie orale ou intraveineuse alors que les compositions selon l'invention sont conditionnées pour être utilisées par voie externe,
- que dans le seul exemple de composition du brevet 48473. dans lequel on utilise de la vitamine B$_6$ la proportion de cette vitamine B$_6$ par rapport au sel de zinc est très faible et que, par conséquent, l'effet potentialisateur mis en évidence dans la présente demande n'est pas obtenu,
- qu'enfin on n'a pas pu démontrer que pour les compositions administrées par voie orale (ou intraveineuse), il pouvait y avoir un effet net du sel de zinc sur l'action de la 5-α-réductase au niveau des follicules pilo-sébacés et encore moins que la vitamine B$_6$ pouvait avoir un effet potentialisateur sur cette activité éventuelle du sel de zinc.

Par ailleurs, le document WO-A-8.404.243 révèle une composition vitaminée complexe contenant des oligo-élément, des métaux et des antibiotiques, sous forme injectable par voie parentérale.

Cette composition antérieure comporte de très nombreux constituants, parmi lesquels on retrouve le zinc et la vitamine $B_6$.

Cependant cette composition antérieure se présente sous une forme mal adaptée à un usage cosmétique ou topique. En outre, cette composition est destinée à favoriser la fécondité chez les ruminants, ce qui constitue une application thérapeutique tout à fait distincte de celle des compositions conformes à la invention.

Les exemples suivants illustrent l'invention.

Exemple 1

On a étudié l'action, in vitro, de sulfate de zinc et de mélanges sulfate de Zinc-vitamine $B_6$ sur la réduction de l'activité de la 5-$\alpha$-réductase. On a utilisé des solutions (pH inférieur à 6) comportant 0,05 %, 0,1 % et 0,5 % de sulfate de zinc ; il a été trouvé que la réduction de l'activité de la 5-$\alpha$-réductase était respectivement de 86 %, 96 % et 98 %. Si l'on ajoute à la solution comportant 0,05 % de sulfate de zinc, 0,05 % de vitamine $B_6$, il a été trouvé que la réduction de l'activité de la 5-$\alpha$-réductase était nettement supérieure à 98 %.

Il apparaît que, grâce à l'utilisation de la vitamine $B_6$, l'action du sulfate de zinc est considérablement potentialisée.

Exemple 2

On a réalisé des solutions aqueuses, non alcooliques, contenant :
- sulfate de zinc 1 % (soit 0,22 % de zinc)
- chlorhydrate de pyridoxine 1 %
et ces solutions utilisées en application sur la peau ou le cuir chevelu ont conduit à une diminution nette de la séborrhée.

Exemple 3

On a réalisé une solution aqueuse contenant O,5 % en poids de sulfate de zinc (soit O,11 % en poids exprimé en zinc métal) et O,5 % en poids de chlorhydrate de pyridoxine.

Cette solution comporte également un conservateur et un parfum. La lotion ainsi obtenue a été appliquée journellement sur le cuir chevelu pendant une durée de quatre mois. On a constaté pour l'essentiel un arrêt de la chute des cheveux et, dans certains cas, une légère repousse de cheveux nouveaux.

Les expérimentations effectuées ont montré que les compositions selon l'invention n'induisaient aucun effet secondaire tel qu'irriration ou allergie.

**Revendications**

1. Compositions cosmétiques ou pharmaceutiques à usage topique, pour la lutte contre les processus alopéciques et/ou séborrhéiques, caractérisées en ce qu'elles contiennent à titre de seul ingrédient actif :
   - de 0,004 à 2 % en poids, exprimé en zinc, d'un sel de zinc
   - de 50 à 1000 % en poids, par rapport au zinc, de vitamine $B_6$.

2. Compositions selon la revendication 1, caractérisées en ce que le sel de zinc est soluble dans l'eau.

3. Utilisation d'une composition comportant de 0,004 à 2 % en poids, exprimé en zinc, d'un sel de zinc, de 50 à 1000 % en poids, par rapport au zinc, de vitamine $B_6$, pour la préparation d'une composition cosmétique ou pharmaceutique destinée à la lutte contre les processus alopéciques et/ou séborrhéiques.

4. Utilisation selon la revendication 3, caractérisée en ce que le sel de zinc précité est soluble dans l'eau.

**Claims**

1. Cosmetic or pharmaceutical compositions for topical use, for combating the alopecic and/or seborrhoeic processes, characterised in that they contain, as the only active ingredient:
   - from 0.004 to 2% by weight, expressed as zinc, of a zinc salt;
   - from 50 to 1000% by weight, relative to the zinc, of vitamin $B_6$.

2. Compositions according to Claim 1, characterised in that the zinc salt is water-soluble.

3. Use of a composition containing from 0.004 to 2% by weight, expressed as zinc, of a zinc salt and from 50 to 1000% by weight, relative to the zinc, of vitamin $B_6$, for the preparation of a cosmetic or pharmaceutical composition intended for combating the alopecic and/or seborrhoeic processes.

4. Use according to Claim 3, characterised in that the abovementioned zinc salt is water-soluble.

**Patentansprüche**

1. Kosmetische oder pharmazeutische Zusammensetzungen zur örtlichen Verwendung für den Kampf gegen die Alopezie-und/oder Seborrhoevorgänge, dadurch gekennzeichnet, daß sie als alleinigen aktiven Bestandteil enthalten:

- von 0,004 bis 2 Gew.-%, als Zink ausgedrückt, eines Zinksalzes;
- von 50 bis 1000 Gew.-%, auf das Zink bezogen, Vitamin $B_6$.

2. Zusammensetzungen nach dem Anspruch 1, dadurch gekennzeichnet, daß das Zinksalz in Wasser löslich ist.

3. Verwendung einer Zusammensetzung, die von 0,004 bis 2 Gew.-%, als Zink ausgedrückt, eines Zinksalzes und von 50 bis 1000 Gew.-%, auf das Zink bezogen, Vitamin $B_6$ aufweist, zur Herstellung einer kosmetischen oder pharmazeutischen Zusammensetzung, die zum Kampf gegen die Alopezie- und/oder Seborrhoevorgänge bestimmt ist.

4. Verwendung nach dem Anspruch 3, dadurch gekennzeichnet, daß das genannte Zinksalz in Wasser löslich ist.